(19) Europäisches Patentamt
European Patent Office
Office européen des brevets

(11) Veröffentlichungsnummer: **0 239 887 A1**

(12) # EUROPÄISCHE PATENTANMELDUNG

(21) Anmeldenummer: **87104089.5**

(22) Anmeldetag: **20.03.87**

(51) Int. Cl.⁴: **C07D 471/14**, A61K 31/435, //(C07D471/14,221:00,221:00,221:00)

(30) Priorität: **22.03.86 DE 3609795**

(43) Veröffentlichungstag der Anmeldung: **07.10.87 Patentblatt 87/41**

(84) Benannte Vertragsstaaten: **AT BE CH DE ES FR GB GR IT LI LU NL SE**

(71) Anmelder: **GÖDECKE AKTIENGESELLSCHAFT**
**Salzufer 16**
**D-1000 Berlin 10(DE)**

(72) Erfinder: **Kleinschroth, Jürgen, Dr.**
**Ricarda-Huchstrasse 11**
**D-7809 Denzlingen(DE)**
Erfinder: **Mannhardt, Karl, Dr.**
**Pfauenstrasse 14**
**D-7807 Elzach-Oberprechtal(DE)**
Erfinder: **Wagner, Bernd, Dr.**
**Am Lossele 5**
**D-7809 Denzlingen(DE)**
Erfinder: **Weinheimer, Günter, Dr.**
**Sachsenstrasse 4**
**D-7809 Denzlingen(DE)**

(54) **Pyrido[4,3-b][1,6]-naphthyridin-derivate, Verfahren zu deren Herstellung und deren Verwendung.**

(57) Die Erfindung betrifft neue Pyrido[4,3-b][1,6]naphthyridin-Derivate der allgemeinen Formel I

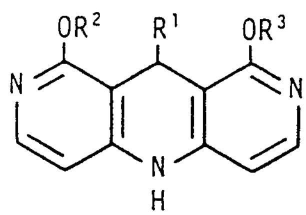

(I)

bzw. deren tautomere Formen der allgemeinen Formeln Ia und Ib

EP 0 239 887 A1

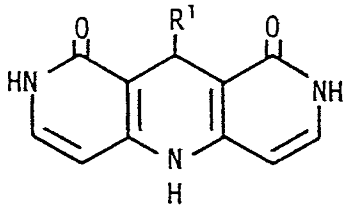

(Ia)

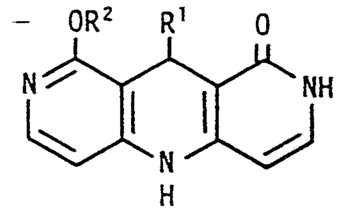

(Ib)

in welchen

$R^1$ einen unsubstituierten oder substituierten aromatischen oder heteroaromatischen Ring oder ein kondensiertes aromatisches oder heteroaromatisches Ringsystem,

$R^2$ und $R^3$ gleich oder verschieden sein können und Wasserstoff oder eine geradkettige oder verzweigte Alkylgruppe mit bis zu 4 Kohlenstoffatomen bedeuten,

sowie gegebenenfalls deren pharmakologisch unbedenkliche Salze.

Die Verbindungen (I) können durch Kondensation von Dihydropyridin-bzw. Naphthyridin-Derivaten mit s-Triazin und ggf. anschließende Alkylierung hergestellt werden und werden zur Bekämpfung von Gefäßerkrankungen verwendet.

## Pyrido[4,3-b][1,6]-naphthyridin-Derivate Verfahren zu deren Herstellung und deren Verwendung

Die Erfindung betrifft neue Pyrido[4,3-b][l,6]naphthyridin-Derivate der allgemeinen Formel I

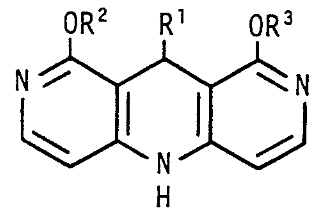

(I)

bzw. deren tautomere Formen der allgemeinen Formeln Ia und Ib

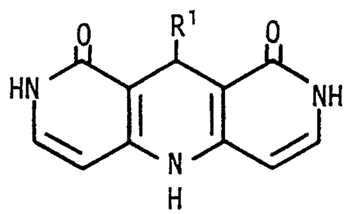

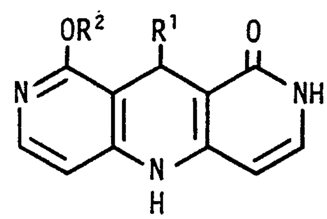

(Ia) (Ib)

in welchen

$R^1$ einen unsubstituierten oder substituierten aromatischen oder heteroaromatischen Ring oder ein kondensiertes aromatisches oder heteroaromatisches Ringsystem,

$R^2$ und $R^3$ gleich oder verschieden sein können und Wasserstoff oder eine geradkettige oder verzweigte Alkylgruppe mit bis zu 4 Kohlenstoffatomen bedeuten,

sowie gegebenenfalls deren pharmakologisch unbedenkliche Salze.

Bevorzugt werden Pyrido[4,3-b][l,6]naphthyridine der allgemeinen Formeln I bzw. Ia oder Ib, in welchen

$R^1$ einen unsubstituierten oder einen vorzugsweise in 2-oder 3-Position durch Halogen, Methoxy, Difluormethoxy, Cyano, Methylthio oder Trifluormethyl substituierten Phenylrest oder einen vorzugsweise in 2,3-durch Methylendioxy oder in 2,3-oder 2,6-Position durch Trifluormethyl oder Halogenatome, die gleich oder verschieden sein können, disubstituierten Phenylrest, einen Naphthyl oder einen 2,l,3-Benzoxadiazolylrest und $R^2$ und $R^3$ Wasserstoff, einen Methyl-, Ethyl-, Propyl-, Isopropyl-, n-Butyl-oder sec-Butylrest bedeuten.

Besonders bevorzugt werden Pyrido [4,3-b][1,6]naphthyridine der allgemeinen Formel I bzw. Ia oder Ib, in welchen

$R^1$ einen unsubstituierten oder einen vorzugsweise in 2-oder 3-Position durch eine Cyano-, Nitro-oder Trifluormethylgruppe substituierten Phenylrest bedeutet.

$R^2$ und $R^3$ Wasserstoff, einen Methyl-, Ethyl-, Propyl-, Isopropyl-, n-Butyl-oder sec-Butylrest bedeuten.

Die Erfindung betrifft weiterhin ein Verfahren zur Herstellung von Pyrido[4,3-b][l,6]naphthyridin-Derivaten der allgemeinen Formeln I bzw. Ia oder Ib, das dadurch gekennzeichnet ist, daß man entweder

a) ein l,4-Dihydropyridin der allgemeinen Formel II

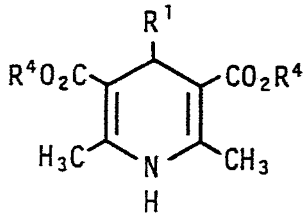

(II)

in welcher $R^1$ die oben angegebene Bedeutung hat, und $R^4$ eine Methyl-oder Ethylgruppe darstellt, in Gegenwart einer Base mit mindestens 2 Äquivalenten s-Triazin umsetzt und die auf diese Weise zunächst erhaltenen Verbindungen der allgemeinen Formel Ia in an sich bekannter Weise bevorzugt zu symmetrisch substituierten Verbindungen der allgemeinen Formel I O-alkyliert,
oder

b) ein l,6-Naphthyridin-Derivat der allgemeinen Formel III,

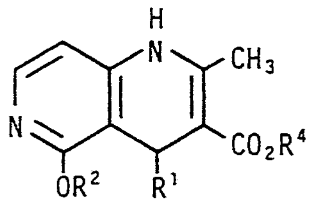

(III)

in welcher $R^1$, $R^2$ und $R^4$ die oben angegebene Bedeutung haben, in Gegenwart einer Base mit mindestens l Äquivalent s-Triazin umsetzt und die auf diese Weise erhaltenen Verbindungen der allgemeinen Formel Ib in an sich bekannter Weise bevorzugt zu unsymmetrisch substituierten Verbindungen der allgemeinen Formel I O-alkyliert.

Die für das Verfahren a) eingesetzten Dihydropyridine der allgemeinen Formel II sind bekannt (vgl. z.B. Chem. Rev. 82 (l982) S. 223) oder sie können in analoger Weise hergestellt werden. Die für das Verfahren b) eingesetzten l,6-Naphthyridine der allgemeinen Formel III sind in DE-OS 34 31 303 beschrieben oder sie sind in analoger Weise zugänglich.

Zur Durchführung der Reaktionen a) und b) wird das l,4-Dihydropyridin-bzw. bzw. das l,6-Naphthyridinderivat mit s-Triazin in einem inerten organischen Lösungsmittel in Gegenwart starker Basen, wie z.B. Alkalialkoholaten oder Natriumhydrid, in einem inerten organischen Lösungsmittel auf Temperaturen von 50-l60°C, vorzugsweise l00-l50°C erhitzt. Als Lösungsmittel eignen sich hier vor allem polare Lösungsmittel wie Dimethylsulfoxid, Dimethylformamid, Ethylenglykoldimethylether oder niedere Alkohole wie Ethanol.

Die Herstellung der Pyrido[4,3-b][l,6]naphthyridine der allgemeinen Formel I bzw. Ib erfolgt erfindungsgemäß nach üblichen, für die O-Alkylierung von Lactamen in der Literatur beschriebenen Verfahren (vgl. Adv. Heterocyclic Chem. l2 (l970), l85-2l2). Geeignete Alkylierungsmittel sind Alkylhalogenide und Alkylsulfonate, Dialkylsulfate und Trialkyloxoniumsalze.

Zur Reaktion mit Alkylhalogeniden werden die Verbindungen der allgemeinen Formeln Ia oder Ib in Form ihrer Metallsalze, vorzugsweise ihrer Alkali-oder Silbersalze eingesetzt, die entweder separat hergestellt oder in situ mit Hilfe geeigneter Basen wie Metallhydriden, -carbonaten oder -alkoxiden in einem aprotischen Lösungsmittel erzeugt werden.

Als geeignete Lösungsmittel kommen, in Abhängigkeit vom jeweiligen Alkylierungsmittel, nahezu alle inerten organischen Lösungsmittel in Frage wie offenkettige, cyclische oder auch aromatische Kohlenwasserstoffe, z.B. n-Pentan, n-Hexan, Cyclohexan, Benzol oder Toluol, halogenierte Kohlenwasserstoffe wie Dichlormethan, l,2-Dichlorethan, Ether wie z.B. Diethylether, l,2-Dimethoxyethan, sowie dipolare aprotische Lösungsmittel wie Dimethylformamid, Hexamethylphosphorsäuretriamid und Dimethylsulfoxid.

Der Temperaturbereich kann je nach verwendetem Lösungsmittel zwischen - 20°C und dem Siedepunkt des betreffenden Lösungsmittels variiert werden.

Aufgrund des ambidenten Charakters des Lactamanions erhält man bei der Alkylierung vielfach Gemische aus O-und N-Alkylierungsprodukten je nach Reaktionsbedingungen und verwendetem Alkylierungsmittel (J. Org. Chem. 32 (l967), 4040 ff).

Die Trennung der erhaltenen Produktgemische kann durch chromatographische Methoden und/oder Kristallisation erfolgen.

Die Pyrido[4,3-b][l,6]naphthyridine der allgemeinen Formel I bzw. Ib werden bevorzugt durch Umsetzung der Verbindungen der allgemeinen Formeln Ia oder Ib mit Trimethyl-bzw. Triethyloxoniumsalzen, insbesondere Trimethyloxoniumtetrafluorborat, in einem aprotischen Lösungsmittel erhalten. Die Herstellung der O-Isopropylverbindungen erfolgt dagegen vorteilhaft durch Alkylierung der Alkalimetallsalze mit Isopropylhalogeniden.

Da die erfindungsgemäßen Verbindungen der allgemeinen Formel I bzw. Ia oder Ib am C-4 ein chirales Zentrum aufweisen können, liegen sie entweder als racemische Gemische oder in Form der Enantiomeren vor.

Die Verbindungen der allgemeinen Formel I bzw. Ia oder Ib sind hochwirksame Calciumantagonisten. Aufgrund ihrer gefäßspasmolytischen Wirkungen sind sie vor allem bei cerebralen, cardialen und peripheren Gefäßerkrankungen indiziert.

Die Pyrido[4,3-][l,6]naphthyridine der vorliegenden Erfindung sind daher wertvolle Arzneimittel für die Bekämpfung der Herz-Kreislauf-Mortalität.

Die erfindungsgemäßen Verbindungen der allgemeinen Formel I bzw. Ia oder Ib können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat-und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäure, höhermolekulare Fettsäuren (wie Stearinsäure), Gelantine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks-und/oder Süßstoffe enthalten.

Die enteral verabreichten Einzeldosen liegen im Bereich von etwa 5 bis 250 mg, vorzugsweise 20 bis 100 mg. Parenteral werden etwa 1 bis 20 mg gegeben.

Ein weiterer Gegenstand der Erfindung sind daher Arzneimittel, enthaltend eine Verbindung der Formeln I, Ia, Ib als Wirkstoff nebst üblichen Hilfs-und Trägerstoffen.

Gegenstand der Erfindung ist auch die Verwendung von Verbindungen der allgemeinen Formeln I, Ia, Ib für die Herstellung von Arzneimitteln zur Bekämpfung von cerebralen, cardialen und peripheren Gefäßerkrankungen.

Die folgenden Beispiele dienen der näheren Erläuterung der Erfindung:

**Beispiel 1**

l,2,5,8,9,l0-Hexahydro-l,9-dioxo-l0-(2-trifluormethylphenyl)pyrido[4,3-b][l,6]naphthyridin

Zu einer Suspension von 6,0 g (0,2 Mol) Natriumhydrid (80%ig in Öl) in 100 ml trockenem Dimethylformamid wird unter Stickstoffatmosphäre eine Lösung von 39,7 g (0,1 Mol) 1,4-Dihydro-2,6-dimethyl-4-(2-trifluormethylphenyl)pyridin-3,5-dicarbonsäurediethylester in 250 ml Dimethylformamid getropft. Nach Abklingen der Gasentwicklung wird noch 10 Minuten bei Raumtemperatur gerührt und anschließend werden 16,2 g (0,2 Mol) s-Triazin in 50 ml Dimethylformamid zugetropft. Die Reaktionsmischung wird 16 Stunden bei 105°C gerührt und nach dem Abkühlen im Vakuum eingeengt. Der Rückstand wird in 200 ml Ethanol aufgenommen und die nach mehrstündigem Rühren im Eisbad ausgefallenen Kristalle abfiltriert. Die Mutterlauge enthält als Nebenkomponente den bereits in P 33 27 650 (Beispiel s) beschriebenen (±)-l,4,5,6-Tetrahydro-2-methyl-5-oxo-4-(2-trifluormethylphenyl)-l,6-naphthyridin-3-carbonsäureethylester. Die Kristalle werden in eine Extraktionshülse gefüllt und 24 Stunden mit Methanol extrahiert. Nach dem Abkühlen kristallisiert aus der Methanollösung l,2,5,8,9,l0-Hexahydro-l,9-dioxo-l0-(2-trifluormethylphenyl)pyrido[4,3-b][l,6]naphthyridin in Form hellbeiger Kristalle vom Schmp. 350°C (Z).

**Beispiel 2**

(±)-I,2,5,IO-Tetrahydro-9-isopropoxy-I-oxo-IO-(2-trifluormethylphenyl)pyrido[4,3-b][I,6]naphthyridin

Zu einer Suspension von I,O g (33mMol) Natriumhydrid (8O%ig in Öl) in 2O ml trockenem Dimethylformamid wird unter Stickstoffatmosphäre eine Lösung von II,3 g (27mMol) (±)-I,4-Dihydro-5-isopropoxy-2-methyl-4-(2-trifluormethylphenyl)-I,6-naphthyridin-3-carbonsäureethylester in 2OO ml Dimethylformamid getropft. Nach Abklingen der Gasentwicklung wird noch IO Minuten bei Raumtemperatur gerührt und anschließend werden 2,5 g (3ImMol) s-Triazin in 2O ml Dimethylformamid zugetropft. Die Reaktionsmischung wird I6 Stunden bei IO5°C gerührt und nach dem Abkühlen im Vakuum eingeengt. Der Rückstand wird an Kieselgel mit Dichlormethan/Methanol 95:5 chromatographiert, die Fraktion mit dem Rf O,6 isoliert und aus Ethylacetat/Ethanol 9:I umkristallisiert.

Man erhält (±)-I,2,5,IO-Tetrahydro-9-isopropoxy-I-oxo-IO-(2-trifluormethylphenyl)pyrido[4,3-b][I,6]-naphthyridin in Form farbloser Kristalle vom Schmp. 3I5°C.

In analoger Weise werden die folgenden Verbindungen erhalten:

(±)-I,2,5,IO-Tetrahydro-9-isopropoxy-I-oxo-IO-phenylpyrido[4,3-b][I,6]naphthyridin (2.a)

Schmp. 299-3OO°C aus Ethylacetat/Ethanol

(±)-1O-(2-Cyanophenyl)-1,2,5,1O-tetrahydro-9-isopropoxy-1-oxo-pyrido[4,3-b][1,6]naphthyridin (2.b)

Schmp. 31O-315°C aus Ethanol

**Beispiel 3**

(±)-5,IO-Dihydro-I-isopropoxy-9-methoxy-IO-(2-trifluormethylphenyl)pyrido[4,3-b][I,6]naphthyridin

7,5 g (I8,7mMol) (±)-I,2,5,IO-Tetrahydro-9-isopropoxy-I-oxo-IO-(2-trifluormethylphenyl)pyrido[4,3-b][I,6]-naphthyridin (siehe Beispiel 2) und 5,O g (34mMol) Trimethyloxoniumtetrafluoroborat werden in 2OO ml I,2-Dichlorethan unter Stickstoffatmosphäre 24 Stunden bei Raumtemperatur gerührt. Dann wird mit gesättigter Kaliumhydrogencarbonatlösung ausgeschüttelt, die organische Phase abgetrennt, mit Wasser gewaschen und über Natriumsulfat getrocknet. Der nach Einengen im Vakuum erhaltene Rückstand wird an Kieselgel mit Toluol/Ethylacetat 3:I chromatographiert. Die Fraktion mit dem Rf O,2 wird isoliert und aus n-Hexan/Diisopropylether umkristallisiert.

Man erhält (±)-5,IO-Dihydro-I-isopropoxy-9-methoxy-IO-(2-trifluormethylphenyl)pyrido[4,3-b][I,6]-naphthyridin in Form farbloser Kristalle vom Schmp. I83-I85°C.

In analoger Weise werden die folgenden Verbindungen erhalten:

(±)-1O-(2-Cyanophenyl)-5,1O-dihydro-1-isopropoxy-9-methoxy-pyrido[4,3-b][1,6]naphthyridin (3.a)

(±)-5,1O-Dihydro-1-isopropoxy-9-methoxy-1O-(3-nitrophenyl)-pyrido[4,3-b][1,6]naphthyridin (3.b)

(±)-1-Sec-Butoxy-5,1O-dihydro -9-methoxy-1O-(2-trifluormethylphenyl)pyrido[4,3-b][1,6]naphthyridin (3.c)

**Beispiel 4**

5,IO-Dihydro-I,9-diisopropoxy-IO-(2-trifluormethylphenyl)pyrido[4,3-b][I,6]naphthyridin

In eine Suspension von O,6 g (2OmMol) Natriumhydrid (8O%ig in Öl) in IOO ml trockenem Dimethylformamid werden portionsweise 3,6 g (IOmMol) I,2,5,8,9,IO-Hexahydro-I,9-dioxo-IO-(2-trifluormethylphenyl)-pyrido[4,3-b][I,6]naphthyridin (siehe Beispiel I) eingetragen, nach Beendigung der Gasentwicklung IO Minuten bei Raumtemperatur gerührt und anschließend eine Lösung von 5,I g (3OmMol) Isopropyljodid in IO ml Dimethylformamid zugetropft. Nach 48 Stunden bei Raumtemperatur wird im Vakuum eingeengt und der Rückstand an Kieselgel mit Toluol/Ethylacetat (3:I) chromatographiert. Die Fraktion mit dem Rf O,4 wird isoliert und aus n-Hexan umkristallisiert.

Man erhält 5,IO-Dihydro-I,9-diisopropoxy-IO-(2-trifluormethylphenyl)pyrido[4,3-b][I,6]naphthyridin in Form farbloser Kristalle vom Schmp. I96-I97°C.

## Ansprüche

1. Pyrido[4,3-b][1,6] naphthyridin-Derivate der allgemeinen Formel I

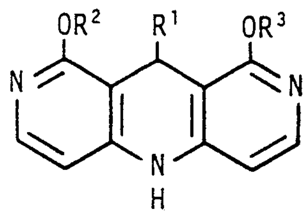

(I)

bzw. deren tautomere Formen der allgemeinen Formeln Ia und Ib

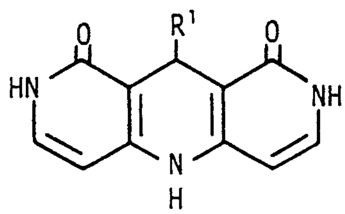

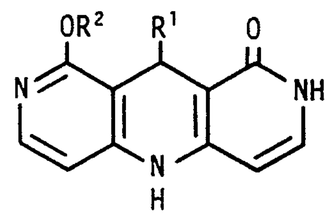

(Ia) (Ib)

in welchen
$R^1$ einen unsubstituierten oder substituierten aromatischen oder heteroaromatischen Ring oder ein kondensiertes aromatisches oder heteroaromatisches Ringsystem,
$R^2$ und $R^3$ gleich oder verschieden sein können und Wasserstoff oder eine geradkettige oder verzweigte Alkylgruppe mit bis zu 4 Kohlenstoffatomen bedeuten,
sowie gegebenenfalls deren pharmakologisch unbedenkliche Salze.

2. Pyrido[4,3-b][1,6]naphthyridin-Derivate nach Anspruch I,
in welchen
$R^1$ einen unsubstituierten oder einen vorzugsweise in 2-oder 3-Position durch Halogen, Methoxy, Difluormethoxy, Cyano, Methylthio oder Trifluormethyl substituierten Phenylrest oder einen vorzugsweise in 2,3-durch Methylendioxy oder in 2,3-oder 2,6-Position durch Trifluormethyl oder Halogenatome, die gleich oder verschieden sein können, disubstituierten Phenylrest, einen Naphthyl oder einen 2,I,3-Benzoxadiazolylrest und
$R^2$ und $R^3$ Wasserstoff, einen Methyl-, Ethyl-, Propyl-, Isopropyl-, n-Butyl-oder sec-Butylrest bedeuten.

3. Pyrido[4,3-b][1,6]naphthyridine der allgemeinen Formeln I bzw. Ia oder Ib nach Anspruch 1 und 2
in welchen $R^2$ die obengenannte Bedeutung hat und
$R^1$ einen unsubstituierten oder einen vorzugsweise in 2-oder 3-Position durch eine Cyano-, Nitro-oder Trifluormethylgruppe substituierten Phenylrest bedeutet.

4. Verfahren zur Herstellung von Pyrido[4,3-b][1,6]naphthyridin-Derivaten der allgemeinen Formeln I bzw. Ia oder Ib, nach Anspruch 1 - 3, dadurch gekennzeichnet, daß man entweder
a) ein 1,4-Dihydropyridinderivat der allgemeinen Formel II,

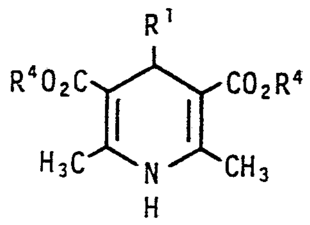

(II)

in welcher $R^1$ die oben angegebene Bedeutung hat, und $R^4$ eine Methyl-oder Ethylgruppe darstellt, in Gegenwart einer Base mit mindestens 2 Äquivalenten s-Triazin umsetzt und die auf diese Weise zunächst erhaltenen Verbindungen der allgemeinen Formel Ia in an sich bekannter Weise bevorzugt zu symmetrisch substituierten Verbindunqen der allgemeinen Formel I O-alkyliert.
oder

b) ein I,6-Naphthyridin-Derivat der allgemeinen Formel III,

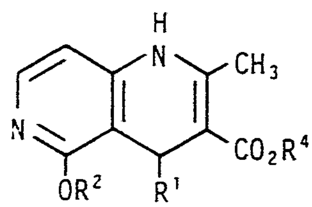

(III)

in welcher $R^1$, $R^2$ und $R^4$ die oben angegebene Bedeutung haben

in Gegenwart einer Base mit mindestens 1 Äquivalent s-Triazin umsetzt und die auf diese Weise erhaltenen Verbindungen der allgemeinen Formel Ib in an sich bekannter Weise bevorzugt zu unsymmetrisch substituierten Verbindungen der allgemeinen Formel I O-alkyliert.

5. Verwendung von Verbindungen nach Anspruch 1 bis 3 für die Herstellung eines Arzneimittels zur Bekämpfung von Gefäßerkrankungen.

6. Arzneimittel, enthaltend eine Verbindung nach Anspruch 1 -3 als Wirkstoff nebst üblichen Hilfs-und Trägerstoffen.

Patentansprüche für folgende Vertragsstaaten : AT, ES, GR

1. Verfahren zur Herstellung von
Pyrido[4,3-b][1,6]naphthyridin-Derivaten der allgemeinen Formel I

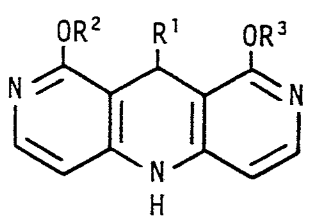

(I)

bzw. deren tautomere Formen der allgemeinen Formeln Ia und Ib

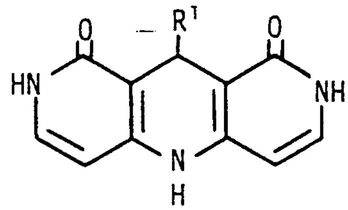

(Ia)

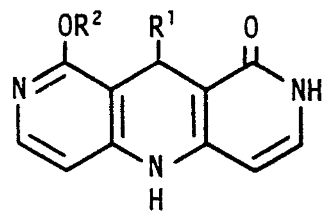

(Ib)

in welchen
$R^1$ einen unsubstituierten oder substituierten aromatischen oder heteroaromatischen Ring oder ein kondensiertes aromatisches oder heteroaromatisches Ringsystem,
$R^2$ und $R^3$ gleich oder verschieden sein können und Wasserstoff oder eine geradkettige oder verzweigte Alkylgruppe mit bis zu 4 Kohlenstoffatomen bedeuten,
sowie gegebenenfalls von deren pharmakologisch unbedenklichen Salzen, dadurch gekennzeichnet, daß man entweder

a) ein1,4-Dihydropyridinderivat der allgemeinen Formel II

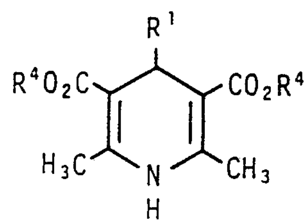

(II)

in welcher $R^1$ die oben angegebene Bedeutung hat, und $R^4$ eine Methyl-oder Ethylgruppe darstellt, in Gegenwart einer Base mit mindestens 2 Äquivalenten s-Triazin umsetzt und die auf diese Weise zunächst erhaltenen Verbindungen der allgemeinen Formel Ia in an sich bekannter Weise bevorzugt zu symmetrisch substituierten Verbindungen der allgemeinen Formel I O-alkyliert.
oder

b) ein l,6-Naphthyridin-Derivat der allgemeinen Formel III,

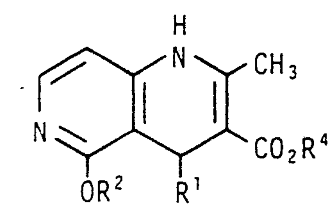

(III)

in welcher $R^1$, $R^2$ und $R^4$ die oben angegebene Bedeutung haben

in Gegenwart einer Base mit mindestens 1 Äquivalent s-Triazin umsetzt und die auf diese Weise erhaltenen Verbindungen der allgemeinen Formel Ib in an sich bekannter Weise bevorzugt zu unsymmetrisch substituierten Verbindungen der allgemeinen Formel I O-alkyliert.

2. Verfahren nach Anspruch 1 zur Herstellung von Pyrido[4,3-b][1,6]naphthyridin-Derivaten der Formeln I, Ia oder Ib,
in welchen
$R^1$ einen unsubstituierten oder einen vorzugsweise in 2-oder 3-Position durch Halogen, Methoxy, Difluormethoxy, Cyano, Methylthio oder Trifluormethyl substituierten Phenylrest oder einen vorzugsweise in 2,3-durch Methylendioxy oder in 2,3-oder 2,6-Position durch Trifluormethyl oder Halogenatome, die gleich oder verschieden sein können, disubstituierten Phenylrest, einen Naphthyl oder einen 2,l,3-Benzoxadiazolylrest und $R^2$ und $R^3$ Wasserstoff, einen Methyl-, Ethyl-, Propyl-, Isopropyl-, n-Butyl-oder sec-Butylrest bedeuten.

3. Verfahren nach Anspruch 1 - 2, zur Herstellung von Pyrido[4,3-b][1-6]naphthyridine der allgemeinen Formeln I bzw. Ia oder Ib in welchen $R^2$ die obengenannte Bedeutung hat und
$R^1$ einen unsubstituierten oder einen vorzugsweise in 2-oder 3-Position durch eine Cyano-, Nitro-oder Trifluormethylgruppe substituierten Phenylrest bedeutet.

4. Verwendung von Verbindungen hergestellt nach Anspruch 1 bis 3 für die Herstellung eines Arzneimittels zur Bekämpfung von Gefäßerkrankungen.

Europäisches Patentamt

# EUROPÄISCHER RECHERCHENBERICHT

Nummer der Anmeldung

EP 87 10 4089

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | WO-A-8 607 359 (SCHERING)<br>* Ansprüche 1,17-19 *<br>----- | 1,5-6 | C 07 D 471/14<br>A 61 K 31/435//<br>(C 07 D 471/14<br>C 07 D 221:00<br>C 07 D 221:00<br>C 07 D 221:00 ) |

| RECHERCHIERTE SACHGEBIETE (Int. Cl.4) |
|---|
| C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 24-06-1987 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument